# EUROPÄISCHE PATENTSCHRIFT

(11) **EP 0 792 867 B1**
(45) Veröffentlichungstag und Bekanntmachung des Hinweises auf die Patenterteilung: **05.06.2002**
(21) Anmeldenummer: 97102813.9
(22) Anmeldetag: 20.02.1997
(51) Int. Cl.: C07C 57/07, C07C 57/05

(54) **Verfahren zur Reinigung von Acrylsäure und Methacrylsäure**
Process for the purification of acrylic and methacrylic acid
Procédé de purification de l'acide acrylique et méthacrylique

(30) Priorität: 23.02.1996 DE 19606877
(43) Veröffentlichungstag der Anmeldung: 03.09.1997
(73) Patentinhaber: BASF AKTIENGESELLSCHAFT, 67056 Ludwigshafen (DE)
(72) Erfinder: Machhammer, Otto, Dr., 68163 Mannheim (DE); Dams, Albrecht, Dr., 67157 Wachenheim (DE); Eck, Bernd, 68519 Viernheim (DE); Proll, Theo, Dr., 67098 Bad Dürkheim (DE)
(74) Vertreter: Isenbruck, Günter, Dr.

(56) Entgegenhaltungen:
- EP-A- 0 616 998
- EP-A- 0 675 100
- DE-A- 2 136 396

## Beschreibung

Die vorliegende Erfindung betrifft ein Verfahren zur Reinigung von Acrylsäure und Methacrylsäure. Außerdem betrifft die Erfindung ein Verfahren zur Herstellung von Acrylsäure oder Methacrylsäure unter Verwendung des Reinigungsverfahrens.

Es ist bekannt, zur Trennung von Stoffgemischen Verfahren einzusetzen, bei denen Phasen vorliegen oder sich bilden. Als Beispiele seien die Extraktion oder Absorption genannt, bei denen Flüssig-/Flüssig- bzw. Gas-/Flüssig-Phasen vorliegen. So beschreibt DE-AS-2 164 767 ein Verfahren zur Reinigung von Acrylsäure, bei dem eine wäßrige Acrylsäurelösung mit einem Extraktionsmittel extrahiert wird, das Extraktionsmittel in einer Destillationszone von dem Extrakt abgetrennt wird und danach in einer Rektifikationszone eine Mischung von Acrylsäure und Essigsäure aus dem verbleibenden Teil des Extraktes abdestilliert wird. DE-C 34 29 391 offenbart ein Verfahren zur Herstellung von Acrylsäure, bei dem das bei der katalytischen Oxidation von Propen und/oder Acrolein erhaltene Gas in einer Acrylsäure-Sammelvorrichtung kondensiert wird und die nicht gewonnenen Substanzen durch Absorption an Wasser gesammelt werden. Die entstehende wäßrige Lösung von Acrylsäure wird dann in einer Destillationskolonne in Anwesenheit eines azeotropen Mittels destilliert, wobei die Acrylsäure im Destillationsbodenprodukt erhalten wird. Ein ähnliches Verfahren ist auch in EP-A-0 551 111 beschrieben.

G.A. Nosov et al. beschreiben in dem Artikel "Separation of binary mixtures by combining rectification and fractionating cristallisation", Russian Chemical Industry, Bd. 25, Nr. 2, 1993, Seiten 6 - 13, die Trennung von Gemischen durch kombinierte Rektifikation und fraktionierte Kristallisation, wobei das zu trennende Gemisch einer Rektifikationsstufe zugeführt wird, und der die Rektifikationskolonne verlassende Dampf einer Kristallisationsstufe zugeführt wird. Dort wird dieser gekühlt, wobei sich eine Kristallphase und Mutterlauge bilden. Nach Abtrennung der kristallinen Fraktion von der Mutterlauge, wird letztere der Rektifikationskolonne rückgeführt. In der Firmenbroschüre "Fraktionierte Kristallisation" der Fa. SULZER CHEM TECH, 1991 ist ebenfalls die kombinierte Anwendung einer Rektifikation und Kristallisation mit Rückführung zur Trennung von Isomerengemischen beschrieben. M. Hassene und G. Drouglazet beschreiben in dem Artikel "The attractions of melt static cristallisation", Chemical Engineering, Sept. 1995, Seiten 108 - 110, die statische Schmelzkristallisation als ein Reinigungsverfahren, das mit einer Destillation kombiniert werden kann. Bei Verfahren zur Herstellung von Naphthalin, Toluolderivaten oder Nitrobenzol wird dieses kombinierte Verfahren zur Endreinigung verwendet. In dem weiteren Artikel "Acrylic acid and acrylates", 91-2, Febr. 1993, CHEM SYSTEMS, Seiten 22 - 26, ist beschrieben, daß durch zweistufige Oxidation hergestellte Roh-Acrylsäure nach Kühlen und Absorption in Wasser durch Lösungsmittelextraktion abgetrennt wird. Die erhaltene weiter aufgereinigte Roh-Acrylsäure wird anschließend einem Reinigungsverfahren unterzogen, wobei es sich um eine Kristallisation handeln kann. Nähere Angaben hierzu enthält dieser Artikel nicht.

Das Japanische Patent 45-32417 offenbart ein Verfahren, bei dem eine wäßrige Acrylsäurelösung bzw. Methacrylsäurelösung, die zusätzlich Essigsäure und Propionsäure enthält, mit Heptan oder Toluol extrahiert wird und anschließend Wasser durch Destillation aus dem Extrakt entfernt wird. In der nächsten Stufe wird der verbleibende Extrakt auf - 20 bis - 80°C abgekühlt, um eine Kristallisation von Acrylsäure oder Methacrylsäure herbeizuführen. Die Kristalle werden abgetrennt, und die Mutterlauge wird dem Extraktionsprozeß rückgeführt. Gemäß dieser Patentschrift ist der Zusatz eines organischen Lösungs- bzw. Extraktionsmittels notwendig, da ansonsten die Lösung, wenn sie abgekühlt wird, sich verfestigt, ohne daß Kristalle ausfallen. In JA-7032417 wird die Reinigung von Methacrylsäure beschrieben, indem diese mit Butadien, Hepten oder Toluol extrahiert wird, der Extrakt durch Destillation dehydratisiert wird und anschließend die Methacrylsäure durch Kühlen auf - 20 bis - 80°C auskristallisiert wird. Die Kristalle werden durch Filtration abgetrennt und das Filtrat rückgeführt. In JA-7110535 wird ein ähnliches Reinigungsverfahren für Acrylsäure beschrieben. Den in den drei zuletzt genannten Druckschriften beschriebenen Verfahren ist gemeinsam, daß die Kristallisation in Anwesenheit eines organischen Lösungsmittels durchgeführt wird.

EP-A 675 100 offenbart ein Verfahren zur Herstellung von α-β-ungesättigten C₃₋₆-Carbonsäuren wie Metharcylsäure oder Acrylsäure. Dabei wird eine entsprechende gesättigte Carbonsäure dehydriert. Das erhaltene Produktgemisch wird fraktioniert destilliert, wobei ein größerer Anteil der gesättigten Säuren über Kopf abgezogen und in den Reaktor zurückgeführt wird. Der verbleibende Sumpf wird einer Kristallisationseinheit zugeführt, in der die ungesättigte Carbonsäure auskristallisiert wird. Die Mutterlauge wird in die Destillationseinheit zurückgeführt.

DE-A 2 136 396 betrifft ein Verfahren zur Herstellung wasserfreier Acrylsäure, das sowohl einen Absorptionsschritt als auch einen Desorptionsschritt umfaßt.

EP-A 616 998 betrifft ein auf fraktionierter Kristallisation basierendes Reinigungsverfahren für Acrylsäure.

Aufgabe der vorliegenden Erfindung war es, ein Verfahren zur Reinigung von Acrylsäure und Methacrylsäure zu schaffen, bei dem bei hoher Reinheit der Säuren eine höhere Ausbeute erzielt werden kann.

Die Aufgabe wird gelöst durch ein Verfahren zur Reinigung von Acrylsäure oder Methacrylsäure, die durch katalytische Gasphasenoxidation von Propen oder Isobuten und/oder Acrolein oder Methacrolein zu Acrylsäure oder Methacrylsäure als gasförmiges, die Säure enthaltendes Reaktionsprodukt erhalten wurde, durch Trennverfahren, bei denen sich Phasen ausbilden, umfassend die folgenden Stufen:
(a) Absorption eines Acrylsäure oder Methacrylsäure enthaltenden gasförmigen Reaktionsprodukts mit einem hochsiedenden Lösungsmittel;
(b) Kondensation des nicht absorbierten Reaktionsgases aus Stufe a) unter Bildung von Sauerwasser und Zurückbleiben von Gas;
(c) Desorption des beladenen Lösungsmittels aus Stufe (a) mit einem Teil des Gases aus Stufe (b) (Strippkreisgas);
(d) Trennung des beladenen Lösungsmittels aus Stufe (c) in das Lösungsmittel und eine Roh-Säure durch Destillation;
(e) Reinigung der Acrylsäure- oder Methacrylsäure aus der Roh-Säure aus Stufe (d) durch Kristallisation;
(f) Rückführung eines Teils der Mutterlauge aus Stufe (g) in die Stufe (a) und/oder Stufe (d).

In einer weiteren Ausführungsform des erfindungsgemäßen Verfahrens wird nach Stufe (d) Stufe (d2) durchgeführt, welche durch das Auswaschen von Leichtsiedern aus dem Strippkreisgas aus Stufe (c) durch in Kontakt Bringen mit einem Teil des Lösungsmittels aus Stufe (d) gekennzeichnet ist.

Außerdem kann auch nach Stufe (d) Stufe (d3) durchgeführt werden, welche durch die Extraktion eines Teils des Lösungsmittels aus Stufe (d) und/oder (d2) mit dem Sauerwasser aus Stufe (b) gekennzeichnet ist.

In einer weiteren Ausführungsform des erfindungsgemäßen Verfahrens wird in Stufe (f) etwa 50% der Mutterlauge in die Stufe (a) und/oder (d) zurückgeführt.

In einer weiteren Ausführungsform des erfindungsgemäßen Verfahrens werden in Stufe (f) etwa 100% der Mutterlauge in Stufe (a) und/oder (d) zurückgeführt.

In einer weiteren Ausführungsform des erfindungsgemäßen Verfahrens wird in Stufe (a) als hochsiedendes Lösungsmittel ein Gemisch aus Diphenylether und Biphenyl verwendet.

In einer weiteren Ausführungsform des erfindungsgemäßen Verfahrens wird die Roh-Säure in Stufe (e) durch dynamische und statische Kristallisation gereinigt.

In einer weiteren Ausführungsform des erfindungsgemäßen Verfahrens wird der Rückstand der dynamischen Kristallation der statischen zugeführt und das Kristallisat der statischen Kristallisation der dynamischen zugeführt.

In einer weiteren Ausführungsform des erfindungsgemäßen Verfahrens wird die dynamische Kristallisation mittels eines voll durchströmten Apparates oder eines Fallfilmkristallisators durchgeführt.

In einer weiteren Ausführungsform des erfindungsgemäßen Verfahrens wird in Stufe (d) Roh-Säure durch Seitenabzug aus der zur Destillation verwendeten Kolonne entnommen.

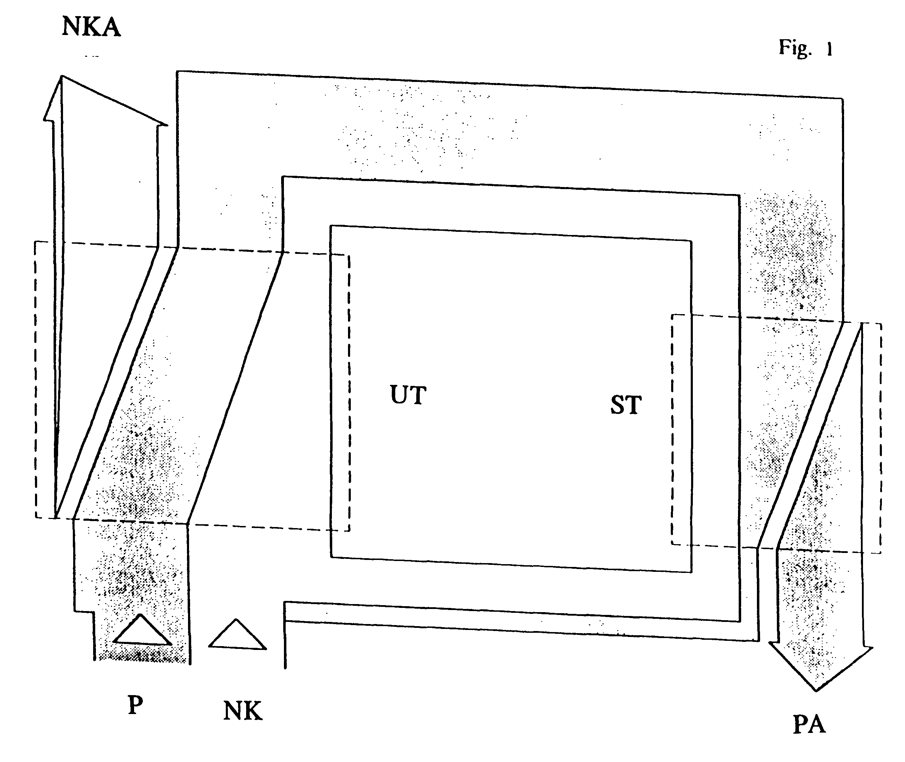

Weitere und bevorzugte Merkmale sind in den Figuren 1 bis 6 und der Beschreibung gezeigt.

In diesem Zusammenhang sind auch die folgenden Überlegungen bedeutsam.

Im Rahmen der Erfindung wird also ein scharfes und ein unscharfes Trennverfahren eingesetzt, wobei die bei dem scharfen Trennverfahren verbleibende, nicht mit Säure angereicherte Phase wenigstens teilweise dem unscharfen Trennverfahren zurückgeführt wird.

Dies geschieht in der Regel durch ein Verfahren zur Reinigung von Acrylsäure oder Methacrylsäure durch Trennverfahren, bei denen sich Phasen ausbilden, wobei
ein die Acrylsäure oder Methacrylsäure enthaltendes Gemisch einem scharfen Trennverfahren im wesentlichen in Abwesenheit eines organischen Lösungsmittels, unterworfen wird, wobei die Phase, in der sich die Acrylsäure oder Methacrylsäure anreichert, in ihrer Zusammensetzung im wesentlichen konstant bleibt, wenn sich die Zusammensetzung der restlichen am Stoffaustausch beteiligten Phasen ändert, diese Phase abgezogen wird, und
(b) die verbleibende Phase von Stufe (a) wenigstens teilweise einem unscharfen Trennverfahren unterworfen wird, und
(c) eine der sich in Stufe (b) bildenden Phasen dem scharfen Trennverfahren in Stufe (a) zugeführt wird.

Das Verfahren zur Herstellung von Acrylsäure oder Methacrylsäure, kann auch folgende Stufen umfassen:
(I) katalytische Gasphasenoxidation von Proben oder Isobuten und/oder Acrolein oder Methacrolein zu Acrylsäure oder Methacrylsäure unter Erhalt eines gasförmigen, die Säure enthaltenden Reaktionsprodukts,
(II) Absorption des Reaktionsprodukts mit einem hochsiedenden Lösungsmittel,
(III) Trennung des beladenen Lösungsmittels aus Stufe (II) durch Destillation in das Lösungsmittel und eine Roh-Säure,
(IV) Reinigung der Acrylsäure oder Methacrylsäure aus der Roh-Säure aus Stufe (III) gemäß dem erfindungsgemäßen Reinigungsverfahren, wobei das unscharfe Trennverfahren die Absorption gemäß Stufe (II) und/oder die Destillation gemäß Stufe (III) ist und das scharfe Trennverfahren eine Kristallisation ist.

In den Zeichnungen zeigen:
- Fig. 1: ein bevorzugtes Ausführungsbeispiel des erfindungsgemäßen Verfahrens zur Reinigung von Acrylsäure oder Methacrylsäure;
- Fig. 2: ein Verfahrensschema zur Herstellung von Acrylsäure bei Verwendung einer statischen und dynamischen Kristallisation;
- Fig. 3: ein Verfahrensschema zur Herstellung von Acrylsäure bei Verwendung einer dynamischen Kristallisation;
- Fig. 4: eine Versuchsanordnung zur Herstellung von Roh-Acrylsäure;
- Fig. 5: eine Versuchsanordnung gemäß Beispiel 1;
- Fig. 6: eine Versuchsanordnung gemäß Beispiel 2.

Bei den gemäß der Erfindung eingesetzten Trennverfahren handelt es sich um Trennverfahren, bei denen sich Phasen ausbilden. Das scharfe Trennverfahren ist gemäß der Erfindung ein Verfahren, bei dem die Phase, in der sich die Acrylsäure oder Methacrylsäure anreichert und/oder in der überwiegend diese Stoffe vorliegen, in ihrer Zusammensetzung im wesentlichen konstant bleibt, wenn sich die Zusammensetzung der restlichen am Stoffaustausch und/oder koexistierenden Phasen ändert. Insbesondere handelt es sich hierbei um ein Trennverfahren, bei dem die Zusammensetzung einer der sich bildenden Phasen im wesentlichen unabhängig von der Zusammensetzung des zugeführten Materials ist. Das scharfe Trennverfahren wird im wesentlichen in Abwesenheit eines organischen Lösungsmittels durchgeführt, vorzugsweise in vollständiger Abwesenheit eines organischen Lösungsmittels. Vorzugsweise enthält das in Stufe (a) zu reinigende Gemisch nicht mehr als 1 Gew. - %, insbesondere nicht mehr als 0,1 Gew.-% organisches Lösungsmittel, jeweils bezogen auf 100 Gew.-% zu reinigendes Gemisch. Das gewählte scharfe Trennverfahren unterliegt dabei keiner Beschränkung. Vorteilhafterweise handelt es sich hierbei um eine Kristallisation, ein Ausfrieren, eine Eindampfung, eine Sublimation ober eine Kombination dieser Verfahren einschließlich dem mehrfachen Einsatz von diesen Verfahren. Am meisten bevorzugt ist die Kristallisation, wobei diese dynamisch und/oder statisch durchgeführt wird.

Besonders bevorzugt ist die dynamische Kristallisation oder eine Kombination von dynamischer und statischer Kristallisation. Bei letzterer Ausführungsform wird, wie in EP-A-0 616 998 beschrieben, vorzugsweise der Rückstand der dynamischen Kristallisation der statischen Kristallisation zugeführt und das Kristallisat der statischen Kristallisation der dynamischen Kristallisation zugeführt. Die Art und Weise der Durchführung der dynamischen und/oder statischen Kristallisation ist hierbei nicht kritisch. Bei der statischen Kristallisation (z.B. US 3 597 164 und FR 2 668 946) wird die flüssige Phase nur durch freie Konvektion bewegt, während bei der dynamischen Kristallisation die flüssige Phase durch erzwungene Konvektion bewegt wird. Letzteres kann durch eine erzwungene Strömung in voll durchströmten Apparaten (vgl. DE-A-2 606 364) oder durch die Aufgabe eines Riesel- oder Fallfilms auf eine gekühlte Wand (DE 1 769 123 und EP-A-0 218 545) erfolgen. Die dynamische und statische Kristallisation können jeweils ein- oder mehrstufig durchgeführt werden. Mehrstufige Verfahren werden hierbei zweckmäßigerweise nach dem Gegenstromprinzip betrieben, bei dem nach der Kristallisation in jeder Stufe das Kristallisat vom Rückstand abgetrennt wird und dieses Kristallisat der jeweiligen Stufe mit dem nächsthöheren Reinheitsgrad zugeführt wird, während der Kristallisationsrückstand der jeweiligen Stufe mit dem nächst niedrigen Reinheitsgrad zugeführt wird. Üblicherweise werden alle Stufen, die ein Kristallisat erzeugen, das reiner ist als die zugeführte Roh-Säurelösung Reinigungsstufen genannt und alle anderen Stufen Abtriebsstufen genannt. Vorteilhafterweise wird die statische Kristallisation in den Abtriebsstufen eingesetzt, und zwar dann, wenn die Ausbeute an der Säure noch weiter erhöht werden soll.

Erfindungsgemäß handelt es sich bei dem unscharfen Trennverfahren um ein Trennverfahren, das nicht unter obige Definition des scharfen Trennverfahrens fällt. Insbesondere handelt es sich hierbei um ein Trennverfahren, bei dem die Zusammensetzung der sich bildenden Phasen abhängig von der Zusammensetzung des zugeführten Materials ist. Die in Frage kommenden unscharfen Trennverfahren unterliegen hierbei keiner besonderen Beschränkung. Zweckmäßigerweise handelt es sich um eine Destillation, Rektifikation, Absorption, Adsorption, Extraktion, Destraktion, ein Membrantrennverfahren, wie eine Pervaporation/Dämpfepermeation, oder eine Kombination dieser Verfahren. Vorteilhafterweise wird eine Destillation, Rektifikation, Absorption oder Extraktion oder eine Kombination dieser Verfahren einschließlich dem mehrfachen Einsatz von diesen Verfahren verwendet. Betrachtet man ein scharfes und ein unscharfes Trennverfahren im Hinblick auf den Trennaufwand und eine Trennstufe, dann zielt bei konstantem Trennaufwand ein scharfes Trennverfahren auf eine höhere Reinheit ab, während ein unscharfes Trennverfahren auf eine höhere Ausbeute abzielt.

Eine besonders vorteilhafte Kombination von scharfen und unscharfen Trennverfahren ist die Kombination von Absorption, Extraktion und/oder Destillation mit Kristallisation.

Das zu reinigende Gemisch oder das Ausgangsmaterial kann erfindungsgemäß jedes beliebige Stoffgemisch sein, das Acrylsäure oder Methacrylsäure enthält. Besonders gut geeignet ist ein Gemisch, wie es bei der Acrylsäureoder Methacrylsäureherstellung anfällt durch Oxidation von Propen bzw. Isobuten, anschließende Absorption mit einem hochsiedenden Lösungsmittel und Destillation oder nach der Oxidation anschließende Kondensation oder Absorption mit Wasser und Extraktion. Solche Gemische enthalten neben der Säure als Verunreinigungen im wesentlichen wenigstens eine der Verbindungen ausgewählt aus Aldehyden, Propionsäure und Essigsäure. Vorzugsweise enthält ein solches Gemisch Acrylsäure oder Methacrylsäure in einer Menge von 90 - 99 Gew.-% und Verunreinigungen vorzugsweise in folgenden Mengen, wobei alle Mengen jeweils auf 100 Gew.-% des Gemisches bezogen sind: Aldehyde 0,05 bis 2 Gew.-%, Propionsäure 0,01 bis 2 Gew.-% und Essigsäure 0,05 bis 2 Gew.-%. Gemische, die in Stufe (a) dem scharfen Trennverfahren unterworfen werden, enthalten im wesentlichen kein organisches Lösungsmittel, vorzugsweise weniger als 1 Gew.-%, insbesondere weniger als 0,1 Gew.-%, besonders bevorzugt weniger als 0,01 Gew.-% organisches Lösungsmittel, jeweils bezogen auf 100 Gew.-% Gemisch.

Gemäß dem erfindungsgemäßen Verfahren wird wenigstens ein Teil der in Stufe (a) verbleibenden, an Acrylsäure oder Methacrylsäure abgereicherten Phase in Stufe (b) dem unscharfen Trennverfahren zugeführt. Das für den jeweiligen Anwendungsfall am besten geeignete Zuführverhältnis kann vom Fachmann leicht im Rahmen fachüblicher Versuche ermittelt werden. Vorzugsweise werden 1 bis 100 Gew.-%, insbesondere 5 bis 50 Gew.-%, am meisten bevorzugt 10 bis 20 Gew.-%, der verbleibenden Phase zugeführt.

In einer vorteilhaften Ausgestaltung der Erfindung wird in Stufe (c) die an Acrylsäure oder Methacrylsäure angereicherte Phase abgetrennt und dem scharfen Trennverfahren in Stufe (a) unterworfen. Es besteht die Möglichkeit, in Stufe (a) und/oder Stufe (b) ein zu reinigendes Ausgangsmaterial, das Acrylsäure oder Methacrylsäure enthält, als Gemisch zuzuführen.

Die vorliegende Erfindung ermöglicht es, durch geeignete Wahl der Menge an von Stufe (a) zu Stufe (b) zugeführter Phase die Ausbeute an der Säure bei im wesentlichen gleichbleibender Reinheit dieses Stoffes entsprechend zu erhöhen.

Fig. 1 zeigt ein bevorzugtes Ausführungsbeispiel des erfindungsgemäßen Verfahrens, bei dem zu reinigendes Ausgangsmaterial vor dem unscharfen Trennverfahren zugeführt wird. Die Dicke der Pfeile zeigt die Mengen an gewünschtem Produkt (Acrylsäure oder Methacrylsäure) und den unerwünschten Nebenkomponenten (NK) an. Die weiteren Bezeichnungen bedeuten: unscharfe Trennung (UT), scharfe Trennung (ST), Produkt (P), Produktabzug (PA), Nebenkomponenten-Ausschleusung (NKA).

In einer bevorzugten Ausführungsform betrifft die Erfindung ein Verfahren zur Herstellung von Acrylsäure oder Methacrylsäure, das die Stufen (I) bis (V) wie oben definiert umfaßt. Die einzelnen Stufen werden im folgenden für Acrylsäure beschrieben. Sie gelten analog für Methacrylsäure, soweit nichts anderes angegeben ist.

### Stufe (I):

Stufe (I) umfaßt die katalytische Gasphasenreaktion von Propen und/oder Acrolein mit molekularem Sauerstoff zu Acrylsäure. Bei Methacrylsäure erfolgt analog eine Gasphasenreaktion von Isobuten und/oder Methacrolein mit molekularem Sauerstoff. Die Gasphasenreaktion kann nach bekannten Verfahren, insbesondere wie sie in den oben genannten Druckschriften beschrieben sind, erfolgen. Vorteilhafterweise wird hierbei bei Temperaturen zwischen 200 und 400°C gearbeitet. Vorzugsweise werden als heterogene Katalysatoren oxidische Mehrkomponenten-Katalysatoren auf der Basis der Oxide von Molybdän, Chrom, Vanadium und/oder Tellur eingesetzt.

Die Umsetzung von Propen zu Acrylsäure ist stark exotherm. Das Reaktionsgas, das neben den Edukten und Produkten vorteilhafterweise ein Verdünnungsgas, z.B. Kreisgas (siehe unten), Luftstickstoff und/oder Wasserdampf enthält, kann daher nur einen kleinen Teil der Reaktionswärme aufnehmen. Deshalb werden als Reaktoren meist Rohrbündelwärmetauscher verwendet, die mit dem Oxidationskatalysator gefüllt sind, und den überwiegenden Teil der bei der Reaktion freiwerdenden Wärme durch Konvektion und Strahlung an die gekühlten Rohrwände abführen.

In Stufe (I) wird jedoch nicht reine Acrylsäure, sondern ein gasförmiges Gemisch erhalten, das Acrylsäure und als Nebenkomponenten im wesentlichen nicht umgesetztes Acrolein und/oder Propen, Wasserdampf, Kohlenmonoxid, Kohlendioxid, Stickstoff, Sauerstoff, Essigsäure, Propionsäure Formaldehyd, weitere Aldehyde und Maleinsäureanhydrid enthalten kann. Insbesondere enthält das Reaktionsproduktgemisch typischerweise, jeweils angegeben in Gew.-% bezogen auf das gesamte Reaktionsgemisch, von 0,05 bis 1 % Propen und 0,05 bis 1 % Acrolein, 0,01 bis 2 % Propan, 1 bis 20 % Wasserdampf, 0,05 bis 15 % Kohlenoxide, 10 bis 90 % Stickstoff, 0,05 bis 5 % Sauerstoff, 0,05 bis 2 % Essigsäure, 0,01 bis 2 % Propionsäure, 0,05 bis 1 % Formaldehyd, 0,05 bis 2 % Aldehyde sowie 0,01 bis 0,5 % Maleinsäureanhydrid.

### Stufe (II)

In Stufe (II) werden die Acrylsäure und ein Teil der Nebenkomponenten aus dem Reaktionsgas durch Absorption mit einem hochsiedenden Lösungsmittel abgetrennt. Hierfür geeignet sind alle hochsiedenden Lösungsmittel, insbesondere Lösungsmittel mit einem Siedepunkt über 160 °C. Besonders geeignet ist ein Gemisch aus Diphenylether und Biphenyl, wie z. B. das im Handel erhältliche Gemisch aus 75 Gew.-% Diphenylether und 25 Gew.-% Biphenyl.

Vorliegend bezeichnen die Begriffe Hoch- oder Schwersieder, Mittelsieder und Leichtsieder sowie entsprechend adjektivisch gebrauchte Begriffe Verbindungen, die einen höheren Siedepunkt als die Acrylsäure besitzen (Hochsieder) bzw. solche, die in etwa den gleichen Siedepunkt wie Acrylsäure besitzen (Mittelsieder) bzw. solche, die einen niedrigeren Siedepunkt als Acrylsäure besitzen (Leichtsieder).

Vorteilhafterweise wird das aus Stufe (I) erhaltene heiße Reaktionsgas durch Teilverdampfen des Lösungsmittels in einem geeigneten Apparat, z.B. einem Direktkondensator oder Quenchapparat, vor der Absorption abgekühlt. Hierfür eignen sich insbesondere Venturiwäscher, Blasensäulen oder Sprühkondensatoren. Dabei kondensieren die schwersiedenden Nebenkomponenten des Reaktionsgases aus Stufe (I) in das nicht verdampfte Lösungsmittel. Außerdem ist die Teilverdampfung des Lösungsmittels ein Reinigungsschritt für das Lösungsmittel. In einer bevorzugten Ausführungsform der Erfindung wird ein Teilstrom des nicht verdampften Lösungsmittels, vorzugsweise 1 bis 10 % des der Absorptionskolonne zugeführten Massenstroms, abgezogen und einer Lösungsmittelreinigung unterworfen. Hierbei wird das Lösungsmittel überdestilliert und zurück bleiben die schwersiedenden Nebenkomponenten, die - bei Bedarf weiter eingedickt - entsorgt, z.B. verbrannt, werden können. Diese Lösungsmitteldestillation dient der Vermeidung einer zu hohen Konzentration an Schwersieder im Lösungsmittelstrom.

Die Absorption erfolgt in einer Gegenstromabsorptionskolonne, die vorzugsweise mit Ventil- oder Dualflowböden bestückt ist, die von oben mit (nicht verdampftem) Lösungsmittel beaufschlagt wird. Das gasförmige Reaktionsprodukt und gegebenenfalls verdampftes Lösungsmittel werden von unten in die Kolonne eingeleitet und anschließend auf Absorptionstemperatur abgekühlt. Die Abkühlung erfolgt vorteilhafterweise durch Kühlkreise, d.h. erwärmtes Lösungsmittel wird aus der Kolonne abgezogen, in Wärmetauschern abgekühlt und wieder an einer Stelle oberhalb der Abzugsstelle der Kolonne zugeführt. In diese Lösungsmittelkühlkreise kondensieren neben der Acrylsäure schwer- und mittelsiedende Nebenkomponenten sowie verdampftes Lösungsmittel. Sobald der Reaktionsgasstrom auf die Absorptionstemperatur abgekühlt ist, erfolgt die eigentliche Absorption. Dabei wird der im Reaktionsgas verbliebene Rest der Acrylsäure absorbiert sowie ein Teil der leichtsiedenden Nebenkomponenten.

Das verbleibende, nicht absorbierte Reaktionsgas von Stufe (I) wird weiter abgekühlt, um den kondensierbaren Teil der leichtsiedenden Nebenkomponenten davon, insbesondere Wasser, Formaldehyd und Essigsäure, durch Kondensation abzutrennen. Dieses Kondensat wird im folgenden Sauerwasser genannt. Der verbleibende Gasstrom, im folgenden Kreisgas genannt, besteht überwiegend aus Stickstoff, Kohlenoxiden und nicht umgesetzten Edukten. Vorzugsweise wird dieser teilweise wieder als Verdünnungsgas den Reaktionsstufen zugeführt.

Aus dem Sumpf der in Stufe (II) eingesetzten Kolonne wird ein mit Acrylsäure, schwer- und mittelsiedenden Nebenkomponenten sowie einem geringen Anteil an leichtsiedenden Nebenkomponenten beladener Lösungsmittelstrom abgezogen und in einer bevorzugten Ausgestaltung der Erfindung einer Desorption unterzogen. Vorteilhafterweise wird diese in einer Kolonne, die vorzugsweise mit Ventil- oder Dualflowböden aber auch mit Füllkörpern oder geordneten Packungen bestückt sein kann, in Anwesenheit eines sog. Stripp- oder Abstreifgases durchgeführt. Bei dem Strippgas kann jedes inerte Gas oder Gasgemisch verwendet werden, vorzugsweise wird ein Gasgemisch von Luft und Stickstoff verwendet, da dieses in Stufe (I) bei Durchführung einer Verdampfung eines Teils des Lösungsmittels anfällt. Bei der Desorption wird der größte Teil der Leichtsieder mit einem Teil des Kreisgases, das vor Stufe (I) entnommen wird, aus dem beladenen Lösungsmittel gestrippt. Da hierbei auch größere Mengen an Acrylsäure mitgestrippt werden, wird zweckmäßigerweise dieser Strom, der im folgenden Strippkreisgas genannt wird, aus wirtschaftlichen Gründen nicht verworfen, sondern rezirkuliert, z. B. in die Stufe, in der die Teilverdampfung des Lösungsmittels erfolgt, oder in die Absorptionskolonne. Da das Strippgas ein Teil des Kreisgases ist, enthält es selbst noch nennenswerte Mengen an Leichtsieder. Die Leistung der zur Desorption verwendeten Kolonne läßt sich erhöhen, wenn man die Leichtsieder vor dem Einleiten in die Kolonne aus dem Strippgas entfernt. Zweckmäßigerweise wird dieses verfahrenstechnisch in der Art durchgeführt, daß man das Strippgas mit in der unten beschriebenen Stufe (III) aufgearbeitetem Lösungsmittel in einer Gegenstromwaschkolonne reinigt.

Aus dem Sumpf der zur Desorption verwendeten Kolonne kann dann ein nahezu leichtsiederfreier, mit Acrylsäure beladener Lösungsmittelstrom abgezogen werden.

### Stufe (III):

In Verfahrensstufe (III) wird die Acrylsäure zusammen mit den mittelsiedenden Komponenten sowie dem letzten Rest an leichtsiedenden Nebenkomponenten vom Lösungsmittel abgetrennt. Diese Trennung erfolgt mittels Destillation, wobei grundsätzlich jede Destillationskolonne verwendet werden kann. Vorteilhafterweise wird hierzu eine Kolonne mit Siebböden, z.B. Dual-Flow-Böden oder Querstromsiebböden aus Metall verwendet. Im Auftriebsteil der Kolonne wird die Acrylsäure vom Lösungsmittel und den mittelsiedenden Nebenkomponenten, wie Maleinsäureanhydrid, frei destilliert. Um den Leichtsiederanteil in der Acrylsäure zu reduzieren, wird vorteilhafterweise der Auftriebsteil der Kolonne verlängert und die Acrylsäure als Seitenabzug aus der Kolonne abgezogen. Diese Acrylsäure wird Roh-Acrylsäure genannt.

Am Kopf der Kolonne wird dann nach einer Partialkondensation ein an Leichtsiedern reicher Strom abgezogen. Da dieser Strom aber noch Acrylsäure enthält, wird er vorteilhafterweise nicht verworfen, sondern der Absorptionsstufe (II) rückgeführt.

Aus dem Sumpf der Kolonne wird das von Leichtsiedern freie und nahezu von Acrylsäure freie Lösungsmittel abgezogen und vorzugsweise zum überwiegenden Teil der Gegenstromwaschkolonne zugeführt, in der das Strippgas von Stufe (II) gereinigt wird, um die Leichtsieder aus dem Strippgas zu waschen. Anschließend wird das nahezu Acrylsäure-freie Lösungsmittel der Absorptionskolonne zugeführt.

In einer bevorzugten Ausführungsform der Erfindung wird das Sauerwasser, das noch Acrylsäure gelöst enthalten kann, mit einem kleinen Teilstrom des nahezu Acrylsäure-freien Lösungsmittels extraktiv behandelt. Bei dieser Sauerwasser-Extraktion wird ein Teil der Acrylsäure in das Lösungsmittel extrahiert und somit aus dem Sauerwasser zurückgewonnen. Das Sauerwasser extrahiert im Gegenzug die polaren mittelsiedenden Komponenten aus dem Lösungsmittelstrom und vermeidet damit eine Zunahme dieser Komponenten im Lösungsmittelkreislauf. Der erhaltene Strom aus Leicht- und Mittelsieder kann, was insbesondere bei Vorhandensein von Umweltschutzauflagen erforderlich sein kann, eingeengt werden.

Die in Stufe (III) erhaltene Roh-Acrylsäure enthält, jeweils bezogen auf die Roh-Acrylsäure, vorzugsweise 98 bis 99,8 Gew.-%, insbesondere 98,5 bis 99,5 Gew.-%, Acrylsäure und 0,2 bis 2 Gew.-%, insbesondere 0,5 bis 1,5 Gew.-% Verunreinigungen, wie z.B. Essigsäure, Aldehyde und Maleinsäureanhydrid. Diese Acrylsäure kann, sofern die Anforderungen an ihre Reinheit nicht sehr hoch sind, gegebenenfalls bereits zur Veresterung verwendet werden.

### Stufe (IV):

Die Abtrennung der Acrylsäure von der aus Stufe (III) erhaltenen Roh-Acrylsäure erfolgt mittels dynamischer oder einer Kombination von dynamischer und statischer Kristallisation, wobei die entstehende Mutterlauge (Restphase) nicht verworfen wird, sondern wenigstens teilweise in die Absorptionsstufe (II) oder Destillationsstufe (III) zurückgeführt wird. Am meisten bevorzugt ist eine Rückführung in die Absorption, da auf diese Weise eine bessere Abtrennung der Nebenkomponenten durch eine Leichtsieder-Wäsche und ein Leichtsieder-Abstreifen möglich ist.

In Stufe (IV) wird die zu reinigende Roh-Acrylsäure in flüssiger Phase in den Kristallisationsapparat eingefüllt und anschließend an den gekühlten Flächen eine feste Phase ausgefroren, die sich in ihrer Zusammensetzung von der eingefüllten flüssigen Phase unterscheidet. Nachdem ein bestimmter Anteil der eingesetzten Acrylsäure ausgefroren ist (zweckmäßigerweise zwischen 50 - 80 %, insbesondere 60 - 70%), wird die verbleibende flüssige Restphase abgetrennt. Vorteilhafterweise erfolgt dies durch einfaches Abfließenlassen oder Abpumpen der Restphase. Dem Kristallisationsschritt können sich weitere Reinigungsschritte wie das sog. Waschen der Kristallschicht (vgl. DE 3 708 709) oder das sog. Schwitzen, d.h. ein partielles Abschmelzen verunreinigter Kristallbereiche, anschließen. Zweckmäßigerweise wird dem Kristallisationsschritt ein Schwitzschritt angeschlossen, wenn die gesamte Reinigungswirkung einer Stufe verbessert werden soll.

Falls gewünscht, kann die in Stufe (IV) erhaltene Rein-Acrylsäure nach bekannten Methoden verestert werden.

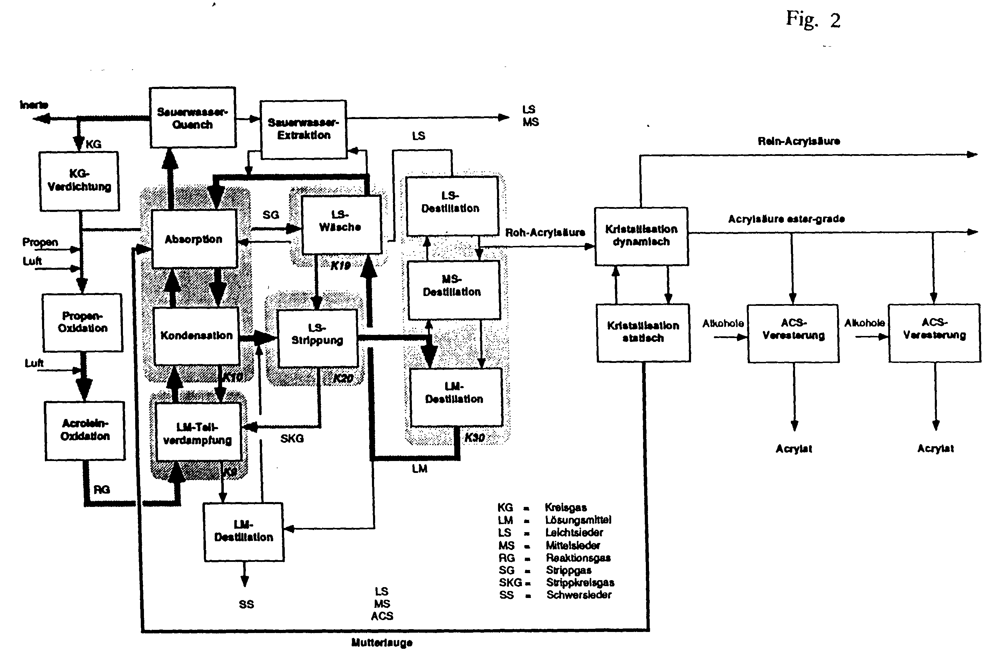

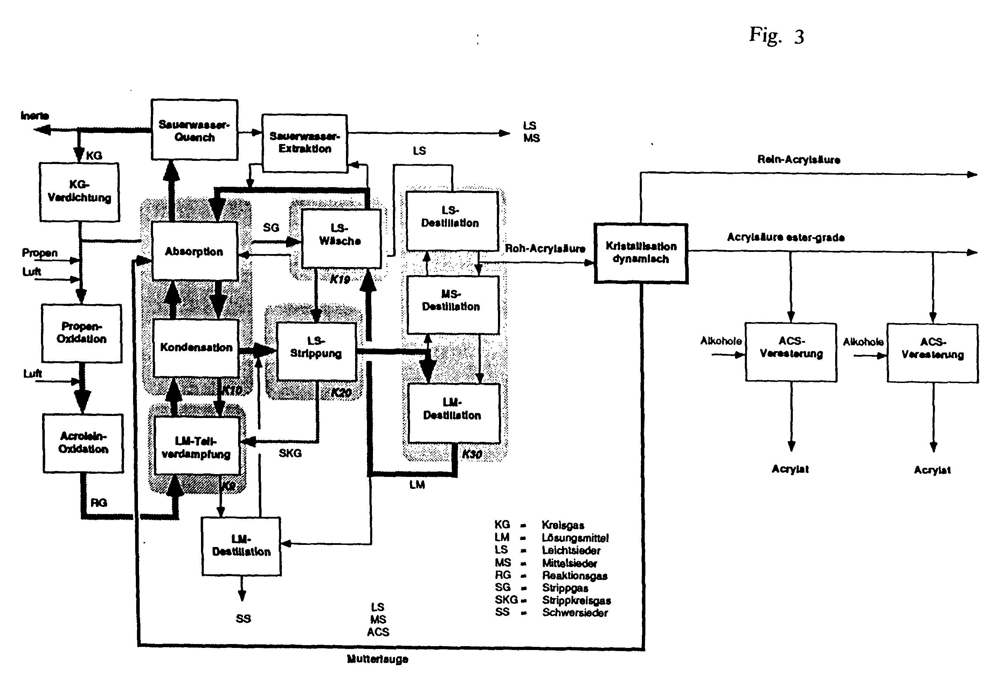

Fig. 2 und 3 zeigen Verfahrensschemata zur Herstellung der Acrylsäure.

Gemäß Fig. 2 wird nach der Verdichtung des Kreisgases, das im wesentlichen aus Stickstoff, Kohlenoxiden und nicht umgesetzten Edukten besteht, dieses zusammen mit Propen und Luft einem Reaktor zugeführt, in dem die heterogen katalysierte Oxidation von Propen zu Acrolein stattfindet. Dem dabei entstehenden Zwischenreaktionsgas wird weiterhin Luft zugeführt, um im zweiten Reaktor die heterogen katalysierte Oxidation von Acrolein durchzuführen.

Das entstehende heiße, die Acrylsäure (ACS) enthaltende, gasförmige Reaktionsprodukt wird durch Teilverdampfen des Lösungsmittels in einem Direktkondensator K9 vor der Absorption abgekühlt. Hierbei kondensieren die schwersiedenden Nebenkomponenten des Reaktionsproduktes in das nichtverdampfte Lösungsmittel. Ein Teilstrom aus dem Direktkondensator K9 wird einer Lösungsmitteldestillation unterzogen, wobei das Lösungsmittel überdestilliert wird und die schwersiedenden Nebenkomponenten zurückbleiben. Letztere können weiter eingedickt und entsorgt, z.B. verbrannt, werden.

Die Kolonne K10, bei der es sich vorzugsweise um eine gefüllte Kolonne handelt, wird von oben mit (nichtverdampftem) Lösungsmittel beaufschlagt, während das verdampfte Lösungsmittel und das gasförmige Reaktionsprodukt von unten in die Kolonne K10 eingeleitet und anschließend auf Absorptionstemperatur abgekühlt werden. Die Abkühlung erfolgt durch Kühlkreise (nicht gezeigt). In diese Kühlkreise kondensieren sowohl das verdampfte Lösungsmittel als auch die Acrylsäure sowie alle schwer- und mittelsiedenden Nebenkomponenten. Nachdem der gesamte Reaktionsgasstrom auf die Absorptionstemperatur abgekühlt ist, erfolgt die eigentliche Absorption. Dabei wird der im Reaktionsgas verbleibende Rest der Acrylsäure absorbiert sowie ein Teil der leichtsiedenden Nebenkomponenten. Anschließend wird das nicht absorbierte, verbleibende Reaktionsgas weiter abgekühlt, um den kondensierbaren Teil der leichtsiedenden Nebenkomponenten aus dem Gasstrom abzutrennen, in Fig. 1 gezeigt als Sauerwasser-Quench. Dieses Kondensat wird Sauerwasser genannt. Der verbliebene Gasstrom, das Kreisgas, kann nun teilweise wieder als Verdünnungsgas den Reaktionsstufen wie in Fig. 1 gezeigt, zugeführt werden.

Aus dem Sumpf der Kolonne K10 wird das mit Acrylsäure und Nebenkomponenten beladene Lösungsmittel abgezogen und der Desorptionskolonne K20 zugeführt. In dieser werden der größte Teil der Leichtsieder mit einem Teil des Kreisgases, das vor den Oxidationsstufen entnommen wird, aus dem beladenen Lösungsmittel gestrippt. Da hierbei auch größere Mengen an Acrylsäure mitgestrippt werden, wird dieser Strom z.B. wieder in den Direktkondensator K9 rezirkuliert.

Zur Erhöhung der Desorptionsleistung der Kolonne K20 werden die Leichtsieder, die im Strippgas enthalten sind, vor dem Einleiten in die Kolonne K20 entfernt. Verfahrenstechnisch zweckmäßigerweise erfolgt dies dadurch, daß das Strippgas mit aufgearbeitetem Lösungsmittel aus der unten näher beschriebenen Kolonne K30 in einer Gegenstromwaschkolonne K19 gereinigt wird.

Im nächsten Verfahrensschritt wird aus dem Sumpf der Desorptionskolonne K20 ein nahezu leichtsiederfreier, mit Acrylsäure beladener Lösungsmittelstrom abgezogen und der Destillationskolonne K30, bei der es sich vorzugsweise um eine Siebbodenkolonne handelt, zugeführt. In den Sumpf der Kolonne K30 kondensiert das schwersiedende Lösungsmittel und die mittelsiedenden Nebenkomponenten, z.B. Maleinsäureanhydrid. Da die am Kopf der Kolonne K30 abgezogene Acrylsäure noch nennenswerte Mengen an leichtsiedenden Nebenkomponenten enthält, reduziert man diesen Leichtsiederanteil zweckmäßigerweise dadurch, daß man den Auftriebsteil der Kolonne K30 weiter verlängert und die Acrylsäure als Seitenabzug aus der Kolonne abzieht. Diese Acrylsäure wird Roh-Acrylsäure genannt.

Der am Kopf der Destillationskolonne K30 abgezogene Leichtsieder-reiche Strom wird, da er noch Acrylsäure enthält, zweckmäßigerweise wieder in die Absorptionskolonne K10 zurückgeführt.

Das aus dem Sumpf der Destillationskolonne K30 abgezogene, Leichtsiederfreie und fast Acrylsäure-freie Lösungsmittel wird zum überwiegenden Teil der Gegenstromwaschkolonne K19 zugeführt, um, wie bereits oben erwähnt, die Leichtsieder aus dem Strippgasstrom, der in die Desorptionskolonne K20 führt, zu waschen. Anschließend wird das nahezu Acrylsäure-freie Lösungsmittel der Absorptionskolonne K10 zugeführt. Mit einem kleinen Teilstrom des nahezu Acrylsäure-freien Lösungsmittels aus dem Sumpf der Destillationskolonne K30 wird das Sauerwasser, das noch Acrylsäure gelöst enthält, extraktiv behandelt. Bei dieser Sauerwasser-Extraktion wird ein Teil der Acrylsäure aus dem Sauerwasser zurückgewonnen, während im Gegenzug das Sauerwasser alle polaren Komponenten aus dem Lösungsmittelteilstrom extrahiert. Das hierbei entstehende Sauerwasser kann vorverdampft und anschließend verbrannt werden.

Die aus dem Seitenabzug der Destillationskolonne K30 gewonnene Roh-Acrylsäure wird anschließend einer dynamischen und statischen Kristallisation unterzogen. Die Mutterlauge aus der statischen Kristallisation wird dann vollständig der Absorptionsstufe zurückgeführt.

Falls gewünscht, wird die erhaltene Rein-Acrylsäure dann mit Alkoholen zu den gewünschten Acrylaten verestert.

Fig. 3 unterscheidet sich von Fig. 2 darin, daß statt der dynamischen und statischen Kristallisation nur eine dynamische Kristallisation vorgesehen ist.

Das erfindungsgemäße Verfahren bietet somit den Vorteil, daß durch Rückführung die Gesamtausbeute an dem gewünschten Stoff und damit die Gesamtwirtschaftlichkeit des Verfahrens erhöht wird. Weiterhin bietet es die Möglichkeit durch Verzicht auf eine statische Kristallisation die Wirtschaftlichkeit nochmals zu erhöhen.

Die Erfindung wird anhand der folgenden Beispiele, die bevorzugte Ausführungsformen der Erfindung darstellen, näher erläutert.

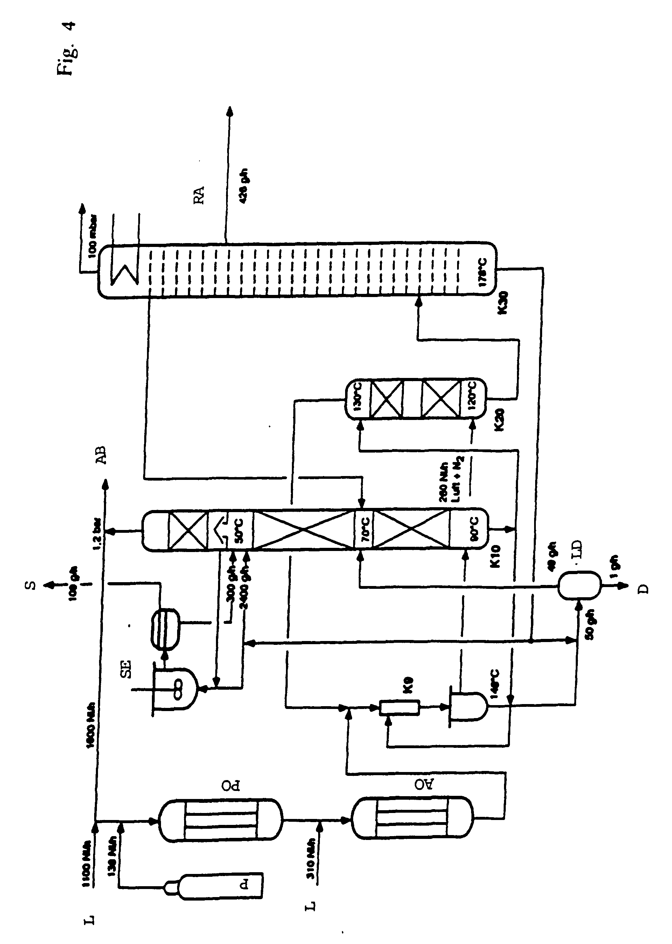

In einer Miniplant wurden stündlich 426 g Roh-Acrylsäure (RA) erzeugt. Die Verschaltung der Apparate, die geförderten Mengen und die eingestellten Betriebsparameter sind in Fig. 4 gezeigt. In dieser Figur sind die gleichen Kolonnen und Apparate wie in Fig. 2 und 3 gezeigt, wobei sich gleiche Bezeichnungen entsprechen (zusätzlich: Lösungsmittel-Destillation: LD; Destillationsrückstand: D, Sauerwasser: S; Sauerwasserextraktion: SE; Abgas: AB). Die Oxidation von Propen (P) mit Luft (L) via Acrolein (A) erfolgte in zwei hintereinandergeschalteten Reaktionsrohren mit 26 mm Durchmesser und 2,5 m Katalysatorschüttungslänge. Das erste Rohr (Propenoxidation: PO) war mit einem Schalen-Katalysator, wie er in EP 575 897 beschrieben ist, gefüllt und das zweite Reaktionsrohr (Acroleinoxidation: AO) enthielt einen Schalen-Katalysator, wie er in EP 609 750 beschrieben ist. Die Kolonnen K10, K20 und K30 waren verspiegelte bzw. thermostatisierte Laborkolonnen mit 50 mm Durchmesser. Der Direktkondensator K9 war ein Venturiwäscher. Die Kolonnen K10 und K20 waren mit 5 mm-Metallwendeln gefüllt. Die Destillationskolonne K30 war mit Siebböden (Dual-Flow-Böden) aus Metall gefüllt. Die Bohrungen in den Siebböden waren so ausgeführt, daß sich Sprudelschichten ausbilden konnten.

### Dynamische Kristallisation

Die dynamische Kristallisation wurde in einem Kristallisator, wie er in DE-A- 26 06 364 (BASF) beschrieben ist durchgeführt, wobei mit einem volldurchströmten Rohr gearbeitet wurde. Die Daten des Kristallisators waren wie folgt:
- zweizügig mit je einem Rohr (Innendurchmesser 26 mm) pro Zug
- Rohrlänge 5 m
- Primärkreispumpe als Kreiselpumpe mit Drehzahlregelung
- Anlagenvolumen primärseitig ca 11 l
- Ausfrierrate ca. 45 % (Ausfrierrate = Kristallisatmasse/Masse Rohschmelze)
- 4 Stufenbehälter mit je 100 l Volumen
- Temperierung der Anlage mit Kälteanlage und 4 bar Dampf über Wärmetauscher

Die Anlage wurde über ein Prozeßleitsystem gesteuert, wobei der Programmablauf für eine Stufe wie folgt war:
1. Füllen des Primärkreises
2. Entleeren des Primärkreises und Anfrieren einer Impfschicht
3. Temperatur auf ca. 2°C unter Schmelzpunkt anheben
4. Primärkreis füllen zum Kristallisieren
5. Kristallisieren (Temperaturprogramm)
6. Nach dem Ende der Kristallisation Restschmelze abpumpen
7. Temperatur anheben zum Aufschmelzen der Kristallschicht
8. Aufgeschmolzenes Kristallisat abpumpen
9. Start einer neuen Stufe

Die Temperaturen, Drücke und Volumenströme sind abhängig von der jeweils zu fahrenden Stufe.

### Statische Schichtkristallisation

Die hierfür verwendete Anlage bestand aus einen Rohrkristallisator aus Glas, der einen Innendurchmesser von 80 mm hatte und dessen Länge 1 m betrug. Der Kristallisator wurde über einen Mantel aus Glas temperiert. Das Füllvolumen des Kristallisators betrug 2,0 bis 5,0 l (variabel). Die Temperierung der Anlage erfolgte über einen Thermostaten, wobei die Temperatursteuerung über einen Programmgeber erfolgte. Die Ausfrierrate (nach dem Schwitzen) betrug ca. 50 %. Der Programmablauf für eine Stufe war wie folgt:
1. Füllen des Kristallisators
2. Temperieren des Apparats mit Inhalt (auf ca. 1 K über Schmelztemperatur)
3. Kristallisieren (Temperaturprogramm)
4. Nach Ende des Kristallisierens Restschmelze ablassen
5. Schwitzen (Temperaturprogramm)
6. Kristallisat abschmelzen
7. Start einer neuen Stufe

Die Temperaturen sind abhängig von der jeweils zu fahrenden Stufe.

Die in den Beispielen 1 bis 3 gezeigten Zahlenwerte ergaben sich aus den tatsächlichen Meßergebnissen einer Mehrzahl von Versuchen.

### Beispiel 1 (Vergleich)

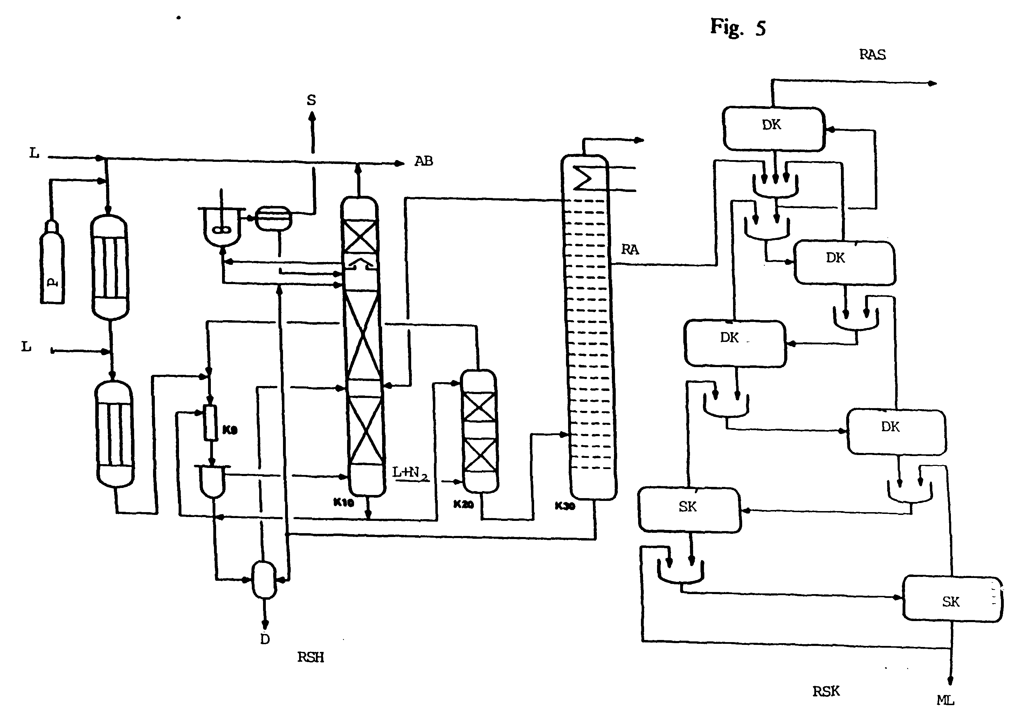

Das Verfahrenschema dieses Beispiels ist in Fig. 5 gezeigt, wobei die Bezeichnungen denen von Fig. 2 bis 4 entsprechen (zusätzlich: dynamische Kristallisation: DK; statische Kristallisation: SK; Mutterlauge: ML; Rein-Acrylsäure: RAS; Rohsäureherstellung: RSH; Reinsäurekristallisation: RSK).

Aus dem Seitenabzug der Destillationskolonne K30 wurden 426 g/h Roh-Acrylsäure in einer Reinheit von 99,7 Gew.-% abgezogen.

Aus der Rohsäureaufarbeitung wurden noch zwei weitere Ströme abgezogen: 109 g/h Sauerwasser mit 3,2 Gew.-% Acrylsäure und 1 g/h Destillationsrückstand mit 2,5 Gew.-% Acrylsäure. Diese beiden Ströme dienen zur Ausschleusung der Nebenkomponenten und wurden deshalb verworfen. Da diese beiden Ströme Acrylsäure enthalten, ist die Ausbeute der Rohsäureaufarbeitung nicht 100 %, sondern lediglich 99,2 %.

Die Roh-Acrylsäure aus der Kolonne K30 wurde anschließend in einer der oben beschriebenen Kristallisationsstufen gereinigt. Dabei wurde eine Rein-Acrylsäure (RAS) in einer Reinheit von 99,95 Gew.-% erhalten. Der Kristallisationsrückstand dieser Reinigungsstufen wurde in 3 dynamischen und 2 statischen Kristallisationsstufen aufgearbeitet. Der Kristallisationsrückstand wurde in diesen 5 Abtriebsstufen auf 4 g/h eingeengt und als sogenannte Mutterlauge mit einem Acrylsäuregehalt von 76,9 Gew.-% aus der Anlage abgezogen und verworfen.

Aufgrund des Acrylsäureverlustes über die verworfene Mutterlauge ist die Ausbeute der Kristallisation nur 99,2 %.

Somit beträgt die Gesamtausbeute 98,4 %.

### Beispiel 2

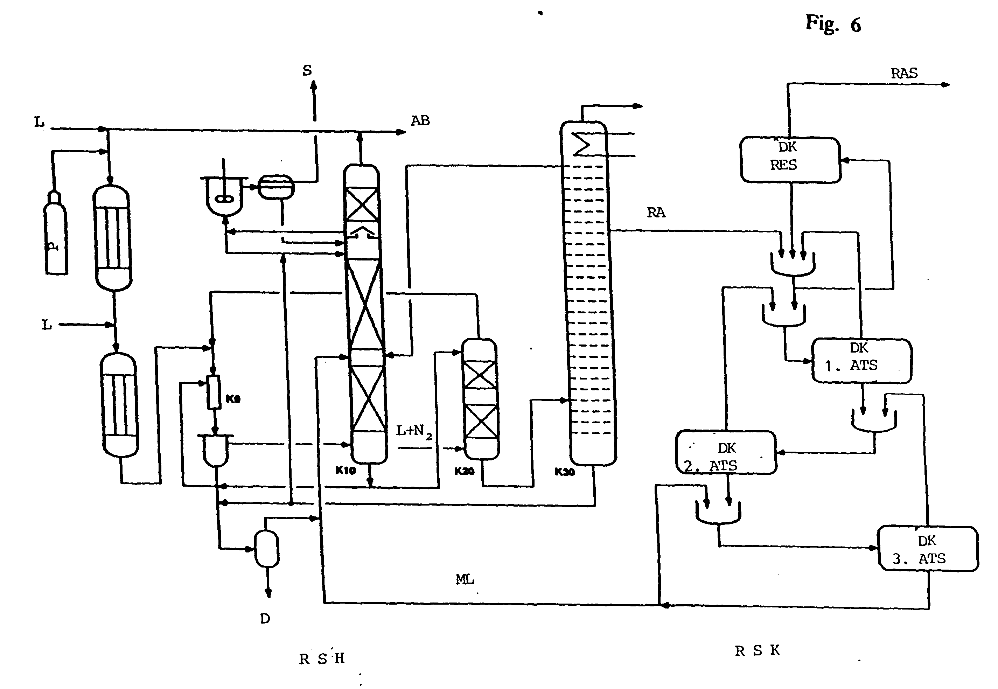

Dieses Beispiel wurde analog zu Beispiel 1 durchgeführt, ausgenommen daß der Kristallisationsrückstand nur in 3 dynamischen Kristallisationsstufen aufgearbeitet wurde und die Mutterlauge aus der Kristallisation nicht verworfen, sondern in die Kolonne K10 (Absorption) vollständig rezirkuliert wurde. Der Verfahrensablauf ist in Fig. 6 gezeigt, wobei die Bezeichnungen denen von Fig. 2 bis 5 entsprechen (zusätzlich: Reinigungsstufe: RES; Abtriebsstufe: ATS).

Aus dem Seitenabzug der Kolonne K30 wurden in diesem Beispiel 579 g/h (nicht 426 g/h) Roh-Acrylsäure in einer Reinheit von 99,7 Gew.-% erhalten.

Aus der Rohsäureaufarbeitung wurden wie in Beispiel 1 109 g/h Sauerwasser mit jedoch 2,9 Gew.-% Acrylsäure und 1 g/h Destillationsrückstand abgezogen. Die Ausbeute der Rohsäureaufarbeitung betrug 99,3 %.

Die Roh-Acrylsäure aus der Kolonne K30 wurde wie in Beispiel 1 gereinigt. Dabei wurde eine Rein-Acrylsäure mit 99,90 Gew.-% Acrylsäure erhalten. Der Kristallisationsrückstand dieser Reinigungsstufe wurde in den 3 dynamischen Abtriebsstufen auf 156 g/h eingeengt. Diese Mutterlauge mit einem Acrylsäuregehalt von 98,9 Gew.-% wurde vollständig in die Kolonne K10 rezirkuliert.

Aufgrund der Rezirkulation der Mutterlauge entsteht in der Kristallisation kein Verlust. Die Gesamtausbeute beträgt somit 99,5 %.

### Beispiel 3

Beispiel 3 wurde analog zu Beispiel 1 durchgeführt, ausgenommen daß 50 % der Mutterlauge aus der Kristallisation in die Kolonne K10 rezirkuliert wurden.

Aus dem Seitenabzug der Destillationskolonne K30 wurden statt 426 g/h jedoch 428 g/h Roh-Acrylsäure in einer Reinheit von 99,7 Gew.-% erhalten.

Aus der Rohsäureaufarbeitung wurden 109 g/h Sauerwasser mit 2,9 Gew.-% Acrylsäure und 1 g/h Destillationsrückstand mit 2,8 Gew.-% Acrylsäure abgezogen. Die Ausbeute der Rohsäureaufarbeitung betrug somit 99,3 %.

Die Roh-Acrylsäure aus der Destillationskolonne K30 hatte nach Reinigung (wie in Beispiel 1) eine Reinheit von 99,95 Gew.-%. Der Kristallisationsrückstand wurde in den drei dynamischen und zwei statischen Abtriebsstufen auf 4 g/h Mutterlauge mit einem Acrylsäuregehalt von 76,1 Gew.-% eingeengt. Hiervon wurde die Hälfte in die Kolonne K10 rezirkuliert und die andere Hälfte wurde verworfen.

Die Gesamtausbeute betrug somit 90,0 %.

Die Ergebnisse der Beispiele 1 bis 3 sind in der folgenden Tabelle zusammengefaßt. In allen Beispielen lag der Gehalt an Propionsäure und Essigsäure in der Reinacrylsäure unter 500 ppm.

**Tabelle**

| Beispiel 1 (Vergleich): | ohne Rückführung der Mutterlauge | |
|---|---|---|
| | Mengenstrom | Acrylsäure |
| **Rohsäureaufarbeitung** | | |
| Roh-Acrylsäure | 426 g/h | 99,7 Gew.-% |
| Verlust Sauerwasser | 109 g/h | 3,2 Gew.-% |
| Verlust Destillationsrückstand | 1 g/h | 2,5 Gew.-% |
| | | |
| Ausbeute Rohsäureaufarbeitung | | 99,2 % |
| | | |

| **Kristallisation** | | |
|---|---|---|
| **Rein-Acrylsäure** | 422 g/h | 99,95 Gew.-% |
| Verlust Mutterlauge | 4 g/h | 76,9 Gew.- |
| | | |
| Ausbeute Kristallisation | | 99,2 % |
| | | |
| **Gesamtausbeute** | | **98,4 %** |
| | | |

| Beispiel 2: | vollständige Rückführung der Mutterlauge | |
|---|---|---|
| | Mengenstrom | Acrylsäure |
| **Rohsäureaufarbeitung** | | |
| Roh-Acrylsäure | 579 g/h | 99,7 Gew.-% |
| Verlust Sauerwasser | 109 g/h | 2,9 Gew.-% |
| Verlust Destillationsrückstand | 1 g/h | 2,8 Gew.-% |
| | | |
| Ausbeute Rohsäureaufarbeitung | | 99,3 % |
| | | |

| **Kristallisation** | | |
|---|---|---|
| **Rein-Acrylsäure** | 425 g/h | 99,90 Gew.-% |
| rezirkulierte Mutterlauge | 156 g/h | 98,9 Gew.-% |
| | | |
| Ausbeute Kristallisation | | 73,5 % |
| | | |
| **Gesamtausbeute** | | **99,5 %** |
| | | |

| Beispiel 3: | teilweise Rückführung der Mutterlauge | |
|---|---|---|
| | Mengenstrom | Acrylsäure |
| **Rohsäureaufarbeitung** | | |
| Roh-Acrylsäure | 428 g/h | 99,7 Gew.-% |
| Verlust Sauerwasser | 109 g/h | 2,9 Gew.-% |
| Verlust Destillationsrückstand | 1 g/h | 2,8 Gew.-% |
| | | |
| Ausbeute Rohsäureaufarbeitung | | 99,3 % |
| | | |

| **Kristallisation** | | |
|---|---|---|
| **Rein-Acrylsäure** | 423 g/h | 99,95 Gew.-% |
| Mutterlauge aus statischer Krist. | 4 g/h | 76,1 Gew.-% |
| rezirkulierte Mutterlauge | 2 g/h | 76,1 Gew.-% |
| | | |
| Ausbeute Kristallisation | | 99,2 % |
| | | |
| **Gesamtausbeute** | | **99,0 %** |

## Patentansprüche

1. Verfahren zur Reinigung von Acrylsäure oder Methacrylsäure, die durch katalytische Gasphasenoxidation von Propen oder Isobuten und/oder Acrolein oder Methacrolein zu Acrylsäure oder Methacrylsäure als gasförmiges, die Säure enthaltendes Reaktionsprodukt erhalten wurde, durch Trennverfahren, bei denen sich Phasen ausbilden, umfassend die folgenden Stufen:
a) Absorption eines Acrylsäure oder Methacrylsäure enthaltenden gasförmigen Reaktionsprodukts mit einem hochsiedenden Lösungsmittel;
b) Kondensation des nicht absorbierten Reaktionsgases aus Stufe a) unter Bildung von Sauerwasser und Zurückbleiben von Gas;
c) Desorption des beladenen Lösungsmittels aus Stufe (a) mit einem Teil des Gases aus Stufe (b) (Strippkreisgas);
d) Trennung des beladenen Lösungsmittels aus Stufe (c) in das Lösungsmittel und eine Roh-Säure durch Destillation;
e) Reinigung der Acrylsäure- oder Methacrylsäure aus der Roh-Säure aus Stufe (d) durch Kristallisation;
f) Rückführung eines Teils der Mutterlauge aus Stufe (g) in die Stufe (a) und/oder Stufe (d).

2. Verfahren nach Aspruch 1, **dadurch gekennzeichnet, daß** nach Stufe (d) Stufe (d2) durchgeführt wird, **gekennzeichnet durch** das Auswaschen von Leichtsiedern aus dem Strippkreisgas aus Stufe (c) **durch** in Kontakt Bringen mit einem Teil des Lösungsmittels aus Stufe (d).

3. Verfahren nach Anspruch 1 oder 2, **dadurch gekennzeichnet, daß** nach Stufe (d) Stufe (d3) durchgeführt wird, **gekennzeichnet durch** die Extraktion eines Teils des Lösungsmittels aus Stufe (d) und/oder (d2) mit dem Sauerwasser aus Stufe (b).

4. Verfahren nach einem der Ansprüche 1 bis 3, **dadurch gekennzeichnet, daß** in Stufe (f) etwa 50% der Mutterlauge in die Stufe (a) und/oder (d) zurückgeführt werden.

5. Verfahren nach einem der Ansprüche 1 bis 3, **dadurch gekennzeichnet, daß** in Stufe (f) etwa 100% der Mutterlauge in Stufe (a) und/oder (d) zurückgeführt werden.

6. Verfahren nach einem der Ansprüche 1 bis 5, **dadurch gekennzeichnet, daß** in Stufe (a) als hochsiedendes Lösungsmittel ein Gemisch aus Diphenylether und Biphenyl verwendet wird.

7. Verfahren nach Anspruch 1, **dadurch gekennzeichnet, daß** die Roh-Säure in Stufe (e) durch dynamische und statische Kristallisation gereinigt wird.

8. Verfahren nach Anspruch 7, **dadurch gekennzeichnet, daß** der Rückstand der dynamischen Kristallation der statischen zugeführt wird und das Kristallisat der statischen Kristallisation der dynamischen zugeführt wird.

9. Verfahren nach Anspruch 7 oder 8, **dadurch gekennzeichnet, daß** die dynamische Kristallisation mittels eines voll durchströmten Apparates oder eines Fallfilmkristallisators durchgeführt wird.

10. Verfahren nach Anspruch 1, **dadurch gekennzeichnet, daß** in Stufe (d) Roh-Säure durch Seitenabzug aus der zur Destillation verwendeten Kolonne entnommen wird.

## Claims

1. A process for purifying acrylic acid or methacrylic acid which has been obtained by catalytic gas-phase oxidation of propene or isobutene and/or acrolein or methacrolein to give acrylic acid or methacrylic acid as a gaseous reaction product comprising the acid, by means of separation processes in which phases are formed, comprising the following steps:
(a) absorption of a gaseous reaction product comprising acrylic acid or methacrylic acid in a high-boiling solvent;
(b) condensation of the reaction gas which has not been absorbed in step (a) to form acidic water and leave gas;
(c) desorption of the loaded solvent from step (a) by means of part of the gas from step (b) (circulated stripping gas);
(d) separation of the loaded solvent from step (c) into the solvent and a crude acid by distillation;
(e) purification of the acrylic acid or methacrylic acid from the crude acid from step (d) by crystalization;
(f) recirculation of part of the mother liquor from step (g) to step (a) and/or step (d).

2. A process as claimed in claim 1, wherein step (d) is followed by step (d2) in which low boilers are scrubbed from the circulated stripping gas from step (c) by bringing the gas into contact with part of the solvent from step (d).

3. A process as claimed in claim 1 and 2, wherein step (d) is followed by step (d3) in which part of the solvent from step (d) and/or (d2) is extracted with the acidic water from step (b).

4. A process as claimed in any of claims 1 to 3, wherein, in step (f), about 50% of the mother liquor is recirculated to step (a) and/or (d).

5. A process as claimed in any of claims 1 to 3, wherein, in step (f), about 100% of the mother liquor is recirculated to step (a) and/or (d).

6. A process as claimed in any of claims 1 to 5, wherein the high-boiling solvent used in step (a) is a mixture of diphenyl ether and biphenyl.

7. A process as claimed in claim 1, wherein the crude acid is purified in step (e) by dynamic and static crystalization.

8. A process as claimed in claim 7, wherein the residue from the dynamic crystalization is passed to the static crystalization and the crystalized material from the static crystalization is passed to the dynamic crystalization.

9. A process as claimed in claim 7 or 8, wherein the dynamic crystalization is carried out by means of an apparatus which is filled by the material flowing through it or by means of a falling film crystalizer.

10. A process as claimed in claim 1, wherein, in step (d), crude acid is taken off via a side offtake from the column used for the distillation.

## Revendications

1. Procédé de purification d'acide acrylique ou d'acide méthacrylique qui a été obtenu par oxydation catalytique en phase gazeuse de propène ou d'isobutène et/ou d'acroléine ou de méthacroléine en acide acrylique ou acide méthacrylique sous la forme d'un produit réactionnel gazeux contenant l'acide, par un procédé de séparation dans lequel des phases se constituent, ce procédé comprenant les étapes suivantes :
a) une absorption d'un produit réactionnel gazeux contenant de l'acide acrylique ou de l'acide méthacrylique par un solvant à point d'ébullition élevé,
b) une condensation du gaz réactionnel non absorbé de l'étape (a) avec formation d'eau acide et subsistance de gaz,
c) une désorption du solvant chargé de l'étape (a) avec une partie du gaz de l'étape (b) (gaz de circuit de stripage),
d) une séparation du solvant chargé de l'étape (c) en le solvant et un acide brut, par distillation,
e) une purification de l'acide acrylique ou méthacrylique provenant de l'acide brut de l'étape (d), par cristallisation,
f) un recyclage d'une partie de la lessive mère de l'étape (e) dans l'étape (a) et/ou l'étape (d).

2. Procédé suivant la revendication 1, **caractérisé en ce que**, après l'étape (d), on effectue une étape (d2), **caractérisée par** le lavage de fractions à bas point d'ébullition à partir du gaz de circuit de stripage de l'étape (c) par mise en contact avec une partie du solvant de l'étape (d).

3. Procédé suivant l'une des revendications 1 et 2, **caractérisé en ce que**, après l'étape (d), on effectue une étape (d3), **caractérisée par** l'extraction d'une partie du solvant de l'étape (d) et/ou (d2) avec l'eau acide de l'étape (b).

4. Procédé suivant l'une des revendications 1 à 3, **caractérisé en ce que**, dans l'étape (f), on recycle environ 50% de la lessive mère dans l'étape (a) et/ou (d).

5. Procédé suivant l'une des revendications 1 à 3, **caractérisé en ce que**, dans l'étape (f), on recycle environ 100% de la lessive mère dans l'étape (a) et/ou (d).

6. Procédé suivant l'une des revendications 1 à 5, **caractérisé en ce que**, dans l'étape (a), on utilise, comme solvant à point d'ébullition élevé, un mélange d'éther diphénylique et de biphényle.

7. Procédé suivant la revendication 1, **caractérisé en ce que** l'acide brut est purifié dans l'étape (e) par cristallisation dynamique et statique.

8. Procédé suivant la revendication 7, **caractérisé en ce que** le résidu de la cristallisation dynamique est amené à la cristallisation statique et **en ce que** le produit de cristallisation de la cristallisation statique est amené à la cristallisation dynamique.

9. Procédé suivant l'une des revendications 7 et 8, **caractérisé en ce que** la cristallisation dynamique est effectuée au moyen d'un appareil totalement traversé ou d'un dispositif de cristallisation à film.

10. Procédé suivant la revendication 1, **caractérisé en ce que**, dans l'étape (d), on prélève de l'acide brut par soutirage latéral à partir de la colonne utilisée pour la distillation.
